(19) Europäisches Patentamt
European Patent Office
Office européen des brevets

(11) Veröffentlichungsnummer : **0 061 641**
**B1**

(12) # EUROPÄISCHE PATENTSCHRIFT

(45) Veröffentlichungstag der Patentschrift : **13.06.84**

(51) Int. Cl.³ : **C 07 C 62/16**, C 07 C 51/04// C07C53/50, C07C51/58, C07C21/04, C07C17/26

(21) Anmeldenummer : **82102075.7**

(22) Anmeldetag : **15.03.82**

(54) **Verfahren zur Herstellung von Caronaldehydsäure und deren Derivaten.**

(30) Priorität : **26.03.81 DE 3111849**

(43) Veröffentlichungstag der Anmeldung : **06.10.82 Patentblatt 82/40**

(45) Bekanntmachung des Hinweises auf die Patenterteilung : **13.06.84 Patentblatt 84/24**

(84) Benannte Vertragsstaaten : **BE CH DE FR GB IT LI NL**

(56) Entgegenhaltungen :
**EP-A- 0 018 533**
**EP-A- 0 021 114**
**FR-A- 2 376 119**
**JOURNAL OF ORGANIC CHEMISTRY, Band 41, Nr. 5, 1976, Seiten 885-887 J.H. BABLER et al.: "Regioselectivity in the cyclization of beta, gamma-epoxy carbanions. Application to the total synthesis of trans-chrysanthemic acid"**
**CESARE FERRI: "Reaktionen der organischen Synthese", G. Thieme Verlag, 1978, Suttgart, DE**

(73) Patentinhaber : **BAYER AG**
**Konzernverwaltung RP Patentabteilung**
**D-5090 Leverkusen 1 Bayerwerk (DE)**

(72) Erfinder : **Arlt, Dieter, Prof. Dr.**
**Rybnikerstrasse 2**
**D-5000 Köln 80 (DE)**

**EP 0 061 641 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von Caronaldehydsäuren und deren Derivaten.

Es sind bereits verschiedene Verfahren zur Herstellung von Caronaldehydsäuren bekannt geworden z. B. durch partielle Reduktion von Cyclopropandicarbonsäurehalbestern mit Diboran oder Natriumborhydrid zum Hydroxymethyl-cyclopropancarbonsäureester der anschließend mit Chromsäure in Pyridin zu Caronaldehydsäure oxidiert wird. Das Verfahren ist jedoch sehr kostspielig, da die verwendeten Ausgangsprodukte nicht leicht zugänglich sind (vgl. DE-OS 2 758 624).

Ein anderes Verfahren geht aus von 4,5-Epoxy-3,3-dimethyl-pentansäureethylester der in absoluten aprotischen Lösungsmitteln mit Litiumdiethylamid zum 2-Hydroxymethyl-3,3-dimethyl-cyclopropancarbonsäureethylester umgesetzt wird, der dann mit Chromsäure in Pyridin zur Caronaldehydsäure oxidiert wird. Auch dieses Verfahren geht von schwer zugänglichen und teuren Ausgangsmaterialien aus (J. H. Babler et al. J. Org. Chem. 41 S. 885 ff (1976)).

Es wurde ein Verfahren zur Herstellung von Caronaldehydsäure und deren Derivaten der Formel I gefunden,

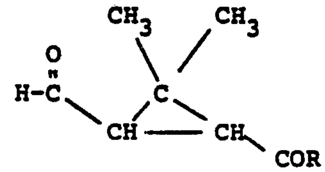

(I)

in welcher

R für $O^-$ $Me^+$, oder OH steht und

$Me^+$ für ein Äquivalent eines Alkali, Erdalkali oder Ammonium-Kations steht

dadurch gekennzeichnet, daß man 2-Halogen-3,3-dimethyl-5,5-dichlor-pentansäurehalogenide der Formel II

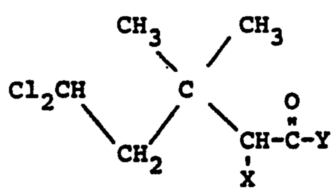

(II)

in welcher

X und Y unabhängig voneinander für Halogen stehen

mit Basen in Gegenwart von Wasser umsetzt und gegebenenfalls die Säure in üblicher Weise freisetzt.

Die Caronaldehydsäure wird unter Umgehung der Stufe der Cyclopropandicarbonsäuren erhalten. Dabei war der Verlauf der Reaktion unter Bildung der Aldehydgruppe überraschend. So war es nach C. Ferri, Reaktionen der organischen Synthese J. Thieme Verlag 1978 S. 202 zu erwarten gewesen, daß eine nicht aktivierte aliphatische Dichlormethyl-Verbindung Chlorwasserstoff unter Bildung einer Doppelbindung abspaltet.

Die erfindungsgemäße Reaktion läßt sich an folgendem Formelschema beispielhaft illustrieren :

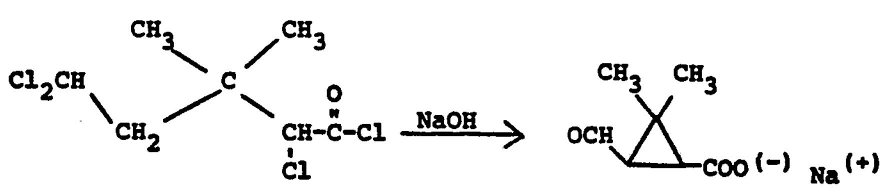


Die als Ausgangsstoffe verwendeten 2-Halogen-3,3-dimethyl-5,5-dichlor-pentansäurehalogenide sind neu. Sie sind Gegenstand einer noch nicht zum Stand der Technik gehörenden Anmeldung der Anmelderin (Le A 20 909). Sie werden erhalten, indem 1,1,1,3-Tetrachlor-3-methyl-butan an Vinylchlorid unter gleichzeitiger Eliminierung von Chlorwasserstoff in Gegenwart von Friedel-Krafts Katalysatoren addiert wird, und das entstandene Vinylidenchlorid in Gegenwart von sauerstoffhaltigen starken Säuren halogeniert wird.

Diese Reaktion läßt sich durch folgendes Reaktionsschema darstellen :

1) 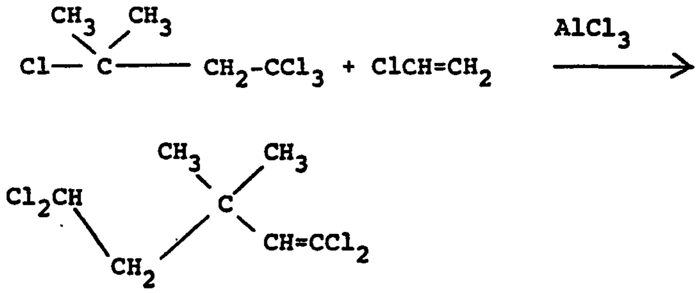


2) 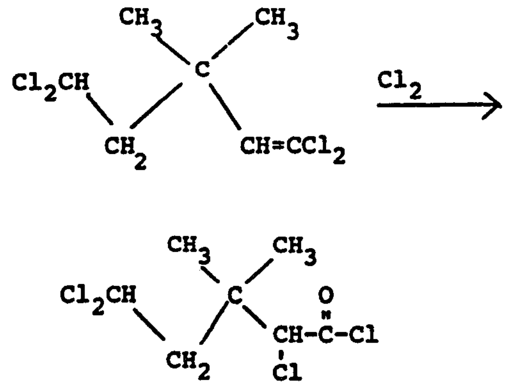


Die erfindungsgemäße Reaktion wird in Gegenwart üblicher Basen wie Alkali- oder Erdalkalihydroxide, tertiäre Amine, Alkali- oder Erdalkalicarbonate durchgeführt.

Als Lösungsmittel dient bevorzugt Wasser. Es kann aber auch in wasserhaltigen mit Wasser nicht mischbaren Lösungsmitteln wie aliphatischen oder aromatischen Kohlenwasserstoffen oder Ethern wie Tetrahydrofuran gearbeitet werden. Als Katalysatoren können übliche Phasentransferkatalysatoren wie Tetralkylammoniumhydroxide zugesetzt werden. Man arbeitet bei Temperaturen von 80-120 °C bevorzugt über 90 °C.

Man arbeitet bei Normaldruck oder leichtem Überdruck. Die Base wird in äquimolarer Menge, gegebenenfalls einem Überschuß von bis zu 10 %, eingesetzt.

Die Aufarbeitung erfolgt in üblicher Weise. Will man die Säure isolieren wird die Salzlösung angesäuert und die Caronaldehydsäure extrahiert. Sie kann durch Destillation gereinigt werden. Die Salzlösung kann aber auch mit Dimethyl- oder Diethylsulfat zum entsprechenden Ester umgesetzt werden.

Die folgenden Beispiele illustrieren die Erfindung ohne eine Begrenzug hinsichtlich ihrer Breite anzugeben :

Beispiel 1

In einem Rührgefäß werden 100 ml Wasser vorgelegt und auf 100 °C erhitzt. Dann werden unter pH-Kontrolle gleichzeitig 63 g (0,25 Mol) 2,5,5-Trichlor-3,3-dimethylpentansäurechlorid und eine Lösung von 58 g NaOH in 100 ml Wasser so zugetropft, daß ein pH-Wert im Bereich von 9-10 eingehalten wird. Nach 30 Minuten ist die Reaktion beendet. Die Reaktionslösung wird auf 20 °C gekühlt, mit Salzsäure auf PH 2 gestellt und mit Dichlormethan extrahiert. Man erhält nach Abtreiben des Lösungsmittels 36 g rohe Säure und nach Destillation im Vakuum 29,6 g (= 83,4 %) reine trans-3-Formyl-2,2-dimethyl-cyclopropan-1-carbonsäure, $Kp._{0,5}$ 125-130.

$^1$H-NMR : δ =1,3 (S, 3H) 1,35 (S, 3H) 2,46 (m, 2H) 9,55 (α, aufgespalten, 1H), 10,95 (S, 1H) ppm.

Herstellung des Ausgangsproduktes :

Beispiel I

In eine Lösung von 15 g $AlCl_3$ in 1 000 ml Methylenchlorid werden 75 g Vinylchlorid bei − 20 °C eingeleitet und dann gleichzeitig 500 g 1,3,3,3-Tetrachlor-1,1-dimethyl-propan der Formel I und weitere 105 g Vinylchlorid zu der Reaktionslösung innerhalb von 180 Minuten zudosiert. Danach läßt man das Reaktionsgemisch 180 Minuten bei − 10° weiterreagieren und versetzt dann die Lösung mit 1 000 ml Wasser. Nach der Trennung wird die wäßrige Phase mehrfach mit Methylenchlorid extrahiert, die vereinigten organischen Phasen werden mit Zeolith getrocknet und franktioniert destilliert. Man erhält 230 g Ausgangsprodukt vom Siedebereich $Kp._{0,1}$ 32-37 °C und 270 g 1,1,5,5-Tetrachlor-3,3-dimethyl-pent-1-en der Formel II vom Siedebereich $Kp._{0,15}$ 72-76 °C. Dieses Ergebnis entspricht einem Umsatz von 54 % mit einer Selektivität von 88 %.

Beispiel II

182 g 1,1,5,5-Tetrachlor-3,3-dimethyl-pent-1-en werden in 400 ml Methansulfonsäure gelöst. Bei 10-20 °C (Wasserkühlung) werden 80 g Chlor eingeleitet. Die Reaktion wird solange weitergeführt, bis eine Probe, die durch Extraktion der Reaktionsmischung mit Hexan erhalten wird, keine IR-Absorption bei 1 610 $cm^{-1}$ zeigt. Dann wird die Reaktionslösung insgesamt mit Hexan extrahiert. Aus der Hexanphase gewinnt man durch Vakuumdestillation — nach Abtreiben des Hexans — 156 g (= 89 % der Theorie) 2,5,5-Trichlor-3,3-dimethyl-pentansäure-chlorid vom Siedebereich $Kp._{0,12}$ 92-95 °C.

**Ansprüche**

1. Verfahren zur Herstellung von Caronaldehydsäure und deren Derivaten der Formel I

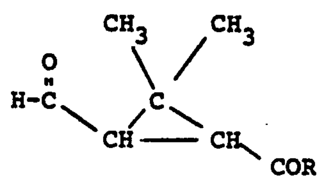

I

in welcher
R für $O^-$ $Me^+$, oder OH steht und
$Me^+$ für ein Äquivalent eines Alkali-, Erdalkali- oder Ammonium-Kations steht
dadurch gekennzeichnet, daß man 2-Halogen-3,3-dimethyl-5,5-dichlor-pentansäurehalogenide der Formel II

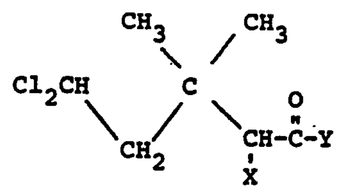

II

in welcher
X und Y unabhängig voneinander für Halogen stehen
mit Basen in Gegenwart von Wasser umsetzt und gegebenenfalls die Säure in üblicher Weise freisetzt.

2. Verfahren gemäß Anspruch 1, dadurch gekennzeichnet, daß in Wasser gearbeitet wird.

**Claims**

1. Process for the preparation of caronaldehyde acid and derivatives thereof of the formula I

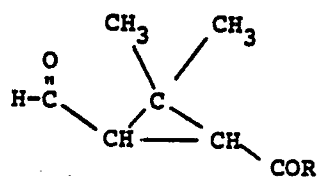

I

wherein
R represents $O^-$ $Me^+$, or OH, and
$Me^+$ represents an equivalent of an alkali metal, alkaline earth metal or ammonium cation,
characterised in that 2-halogeno-3,3-dimethyl-5,5-dichloropentanoic acid halides of the formula II

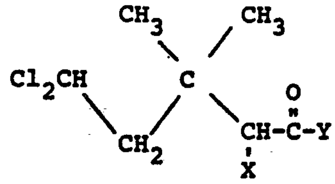

II

wherein

X and Y independently of one another represent halogen,

are reacted with bases in the presence of water, and the acid is liberated, if appropriate, in the customary manner.

2. Process according to Claim 1, characterised in that the reaction is carried out in water.

**Revendications**

1. Procédé de préparation d'acide caronaldéhydique et de ses dérivés de formule I

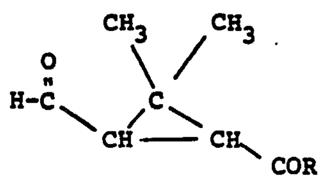

I

dans laquelle

R représente $O^-$ $Me^+$ ou OH, et

$Me^+$ représente un équivalent d'un cation alcalin, alcalino-terreux ou ammonium,

caractérisé en ce qu'on fait réagir des halogénures d'acides 2-halogéno-3,3-diméthyl-5,5-dichloropentanoïques de formule II

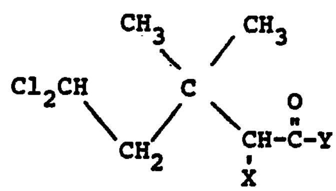

II

dans laquelle

X et Y représentent chacun indépendamment l'un de l'autre un atome d'halogène,

avec des bases en présence d'eau et on libère éventuellement l'acide de la manière habituelle.

2. Procédé suivant la revendication 1, caractérisé en ce qu'on travaille dans l'eau.